(19) **Europäisches Patentamt**

**European Patent Office**

**Office européen des brevets**

(11) **EP 1 256 349 A1**

(12) **EUROPEAN PATENT APPLICATION**
published in accordance with Art. 158(3) EPC

(43) Date of publication:
**13.11.2002 Bulletin 2002/46**

(21) Application number: **01904441.1**

(22) Date of filing: **14.02.2001**

(51) Int Cl.[7]: **A61K 35/64**, A61K 31/7016, A61K 7/00, A61K 7/48, A61P 43/00, A61P 7/00, A23L 1/29, A23L 1/076

(86) International application number:
**PCT/JP01/01045**

(87) International publication number:
**WO 01/060388 (23.08.2001 Gazette 2001/34)**

(84) Designated Contracting States:
**AT BE CH CY DE DK ES FI FR GB GR IE IT LI LU MC NL PT SE TR**

(30) Priority: **15.02.2000 JP 2000037200**

(71) Applicant: **Kabushiki Kaisha Hayashibara Seibutsu Kagaku Kenkyujo Okayama-shi, Okayama 700-0907 (JP)**

(72) Inventor: **MIYAKE, Tos., Hayashibara Seibutsu Kagaku Kenkyujo Okayama-shi, Okayama 700-0907 (JP)**

(74) Representative: **Daniels, Jeffrey Nicholas et al Page White & Farrer 54 Doughty Street London WC1N 2LS (GB)**

(54) **CELL ACTIVATOR**

(57)   The object of the present invention is to provide a daily usable manes for exerting the tonic action inherent to royal jellies, and is solved by providing a cell activating agent comprising a royal jelly and trehalose.

**Description**

TECHNICAL FIELD

The present invention relates to a novel cell activating agent, more particularly, to a cell activating agent comprising a royal jelly and trehalose.

BACKGROUND ART

Royal jelly is a white milky secretion from exocrine glands of worker bees, which is accumulated in the royal cell in bees' nests and fed to a larva to be grown up into a queen bee. The larva, that is undistinguishable from other worker bees when it ecloses in the royal cell, grows into a queen bee that has a larger size and a longer life-span than those of worker bees, as well as having a high egg-productivity after having been grown up on a sufficient amount of royal jelly. Based on this, royal jelly has been recognized to have a variety of physiological functions such as tonicity and promotion of stamina. For example, it is described in *Shikou Bessatsu-Honey-Book* (titled *"Preference"* in special edition of "*Meidi-ya's Honey Book"*), page 22, published by Meidi-Ya Honsha, 1965, that "*Royal jelly may have distinctive medical effects such that it augments physical strength and stamina, improves skin activity, and turns gray hairs into black".* Since royal jelly is a natural product, it would be substantially free from serious side effect if taken by mammals including humans. Based on these, the application of royal jelly for exerting tonicity has been greatly expected that it would overcome unfavorable side effect frequently found in conventional tonics.

However, royal jelly has the drawback that it easily deteriorates when in an intact form just after collection and then promptly loses its inherent actions. To overcome the defect, royal jelly is preserved at temperatures lower than $0°C$ or at lower temperatures thereabout or used after dryness. These treatments may exert a comparatively effective inhibitory action on quality deterioration of royal jelly during storage, but have troublesome in its handling during preservation at lower temperatures. The dryness of royal jelly would inevitably deteriorate the inherent quality of royal jelly during its processing, and this hinders sufficient exertion of the inherent action of royal jelly. As described above, in spite of its expectation, royal jelly could not be said to have been being used as a tonic easily and effectively.

In view of the above backgrounds, the object of the present invention is to provide a means for exerting the tonic action inherent to royal jellies and being used daily with ease.

DISCLOSURE OF INVENTION

To solve the above object, the present inventors compared and studied the action of compositions, which had been prepared by incorporating different substances into an intact royal jelly that had had been preserved at a relatively low temperature ("intact royal jelly" may be designated as "raw royal jelly", hereinafter), and then preserved at various conditions for a prescribed period of time, where a raw royal jelly after preservation at a relatively low temperature was used. As a result, the present inventors found by themselves that the compositions, which had been incorporated with royal jelly and trehalose as in the above, stably retained the cell activating activity of royal jelly even after the preservation at ambient temperature conditions for a relatively long period of time, and would quite effectively strengthen mammals including humans. The present invention was made based on the above finding.

The present invention solves the above object by providing a cell activating agent comprising a royal jelly and trehalose, process for producing the same, and uses thereof.

BEST MODE FOR CARRYING OUT THE INVENTION

The cell activating agent of the present invention comprises a royal jelly and trehalose. The term "royal jelly" as referred to as in the present invention means a white milky secretion from exocrine glands of worker bees, which is accumulated in the royal cell in bees' nests and fed to a larva to be grown up into a queen bee, and is not specifically limited to particular species of bees that secrete royal jelly and to the production region of royal jelly. The species of bees that secrete royal jelly include *Apis mellifera, Apis cerana, Apis dorsata, Apis florea,* etc. The production countries of royal jelly are, for example, Japan, South America, North America, Australia, China, and Europe. In general, royal jelly may induce unfavorable affects such as allergic reactions depending on subjects to be applied. Royal jellies from South America, particularly, those from Brazil are comparatively low in such affects, and therefore the above products can be advantageously used in the present invention. As materials for use in the present invention, preferably used are raw royal jellies as fresh as possible or those preserved under cold conditions.

The trehalose as referred to as in the present invention means $\alpha,\alpha$-trehalose composed of two moles of glucose linked together at their reducing groups. Since trehalose effectively stabilizes the cell activating activity of royal jelly, the saccharide is advantageously used in the present invention. In the cell activating agent of the present invention, there is no restriction of preparation, purity, and properties of trehalose used in the present invention as long as the saccharide is incorporated into the agent in an amount effective to stably retain the cell activating activity of royal jelly. Thus, any of hydrous and anhydrous trehaloses, saccharide compositions comprising either or both of these types of trehaloses, and crystals of either of these trehaloses is arbitrarily used in the present invention. Anhydrous trehalose is relatively preferably used when the cell activating agent of the present invention is provided in a solid form, for example, a powder, tablet, or block.

The trehalose usable in the present invention can be prepared by different methods: For example, in view of economical viewpoint, the methods where a non-reducing saccharide-forming enzyme and a trehalose-releasing enzyme are allowed to act on partial starch hydrolyzates, as disclosed in any of Japanese Patent Kokai Nos. 143,876/95, 213,283/95, 322,883/95, 298,880/95, 66,187/96, 66,188/96, 336,388/96, and 84,586/96, applied for by the same applicant as the present invention, can be preferably used. According to the above method, trehalose can be obtained in a higher yield from low-cost material starches. Examples of commercialized products prepared by the above method include "TREHA®", a crystalline trehalose hydrate with a trehalose content of at least 98%, on a dry solid basis (d.s. b.); and "TREHASTAR®", a trehalose syrup with a trehalose content of at least 28%, d.s.b., which are commercialized by Hayashibara Shoji Inc., Okayama, Japan. Also, trehalose can be prepared by subjecting maltose to the action, for example, of a maltose/trehalose converting enzyme as disclosed in Japanese Patent Kokai No. 170,977/95, 263/96, or 149,980/96 applied for by the same applicant as the present invention; or to the action of conventional maltose phosphorylase and trehalose phosphorylase in combination. Anhydrous trehalose can be prepared, for example, by drying the crystalline trehalose hydrate obtained in the above at temperatures of 70 to 160°C under normal or reduced pressures, preferably, at temperatures of 80 to 100°C under reduced pressures; or by placing in a crystallizer a high trehalose content solution with a moisture content of less than 10%, stirring the solution in the presence of a seed crystal at 50 to 160°C, preferably, 80 to 140°C, to prepare a massecuite containing anhydrous crystalline trehalose; and crystallizing trehalose from the massecuite and pulverizing the resulting crystals by block pulverization, fluidized-bed granulation, spray drying, etc., under relatively high temperatures and drying conditions.

The cell activating agent of the present invention comprises the above-mentioned royal jelly and trehalose. The ratio of royal jelly and trehalose in the cell activating agent includes those which attain the cell activating activity and the stabilization of royal jelly. The cell activating action of royal jelly can be confirmed according to the method in the later described experiments. While the stabilization of the cell activating activity of royal jelly can be confirmed by comparing the residual percentages (%) of one or more of the cell activating actions as indexes, after storing under a prescribed condition. The cell activating agent in a solid form of the present invention, which comprises, to the total amount, at least one percent (%) by weight, preferably, at least two percent (%) by weight of a raw royal jelly and, in terms of anhydrous form, trehalose in an amount of 0.2 time by weight, preferably, at least two times by weight of the raw royal jelly, has characters that it has both a particularly superior, remarkable cell-life prolonging action and another action of activating the function of testis, as examples of cell activating actions, and also has a satisfactory taste. From a viewpoint of the stability of cell activating action and the taste of the agent, there is no upper limit of the ratio of trehalose to royal jelly in the cell activating agent of the present invention, however, the ratio should preferably be, usually, 50 times or lower, desirably, 25 times or lower of the royal jelly by weight in terms of raw royal jelly, when another ingredients are further incorporated into the agent as described later.

The addition of an antioxidant to the cell activating agent of the present invention thus obtained will stabilize the cell activating action of the agent. Therefore, depending on subjects/symptoms or regions to be applied or used, antioxidants are advantageously, optionally added to the cell activating agent, for example, when transported by ships without thermal control or used in hot regions. The antioxidants usable in the present invention are not restricted to specific ones, however, they are preferably selected from those which are generally used in the field of food products, where the cell activating agent is used as a food for mammals including humans. Examples of antioxidants used conventionally in the filed of foods include flavonoids, polyphenols, vitamin E, and vitamin C. Particularly, the above flavonoids are, for example, rutin, hesperidin, naringin, quercetin, and saccharide-transferred products thereof to which bind a saccharide such as glucose and polymers thereof. Examples of the above polyphenols include catechin, gallic acid, and proanthocyanidin. In addition, plant extracts such as *sophora*, rose marry, *Eucalyptus*, and grape seeds can be arbitrarily used as antioxidants in the present invention. Although the amount of these antioxidants to be incorporated into the cell activating agent of the present invention is not specifically restricted, it is preferably chosen according to conventional ratios used in the field of food products or depending on ratios lower than the above, in view of the influence of the agent on taste of foods when applied thereunto.

When in a sold form, the cell activating agent of the present invention is satisfactory stable and, as compared with conventional royal jellies, is characteristic in that it stays longer in the oral cavity when orally taken. This means that the cell activating agent of the present invention is easily absorbed directly by living bodies via the oral mucosa/skin without assimilation in their digestive systems. Thus, the cell activating agent will exert the desired effect in a lesser amount as compared with conventional royal jelly. Since sugar alcohols such as maltitol and viscous polymers such as pullulan do not affect the quality of royal jelly and promote the staying within the oral cavity, the sugar alcohols and/or the viscous polymers can be arbitrarily incorporated into the agent.

If necessary, into the cell activating agent of the present invention can be arbitrarily incorporated another ingredients, excluding the above ones, such as emulsifiers, flavors, seasonings, pigments, vitamins, amino acids, and minerals. These another ingredients can be appropriately selected from those which are generally used in different fields to which the cell activating agent can be applied, depending on purpose. The cell activating agent containing any of the above ingredients is not restricted to have a specific form and can be provided in the desired form such as a powder, tablet, film, sheet, paste, milky lotion, or liquid.

The cell activating agent thus prepared will effectively exert one or more of the cell activating actions inherent to royal jelly for a relatively long period of time. The above cell activating actions are, for example, survival extension of cells such as liver cells, promotion of proliferation or differentiation of cells such as immunocompetent cells, augmentation of biological defence functions of immunocompetent cells, promotion of cell metabolism, prevention of aging of reproductive center cells, and activation of testis function. Because of the above cell activating actions, royal jelly may result in an exertion of inhibiting the aging associated increase of total cholesterols and/or total lipids in serum. These cell activating actions by royal jelly can be confirmed experimentally by phenomena such as survival extension of cells cultured *in vitro,* augmentation of DNA synthesis in culture cells, inhibition of apoptosis of cells, activation of cells relating to the function of orchis such as seminiferous tubules of experimental animals, and life extension of the animals. Varying depending on the kinds/types of cell activating actions to be compared, as well as ingredients, compositions, and preservation conditions of the cell activating agent, the residual percentage (%) of intact royal jelly is usually less than 40% when stored at 25°C for 30 days, however, when formulated into the cell activating agent according to the present invention, the residual percentage (%) will increase to, usually, at least about 50%, preferably, at least 60%, and more preferably, at least 80%.

Since the cell activating agent of the present invention stably exerts the above cell activating actions, it effectively activates and suppresses the lowering of functions of each cell in mammals including humans when taken orally. Thus, the cell activating agent of the present invention can be applied to those, which are easily applied to mammals including humans and to maintain/promote the health, for example, tonics, health foods, and health supplements. Varying depending on the family/genus/species, age and gender of such mammals, as well as on the expected effects, the cell activating agent is allowed to be taken or administered to the mammals at a dose, usually, of 0.1 mg to 10 g per kg body weight, preferably, 1 mg to 1 g per day one to several times a day for consecutive days or at an interval of one or more days, depending on the effect.

To prepare the cell activating agent of the present invention, based on the ingredients and on the amount of each ingredient appropriately selected depending on purpose, particularly, based on mammals to be treated and the route for intake or administration, the ingredients are mixed together and optionally, appropriately diluted, concentrated, dried, filtered, centrifuged, etc., followed by collecting portions/fractions containing royal jelly and trehalose as the ingredients of the cell activating agent of the present invention and, if necessary, further forming the resultant mixture into the desired shape. The order of incorporation of each ingredient and the timing for applying the above treatments are not specifically restricted as long as they prevent the deterioration of the quality of royal jelly: For example, trehalose is mixed with either a raw royal jelly as fresh as possible or one which has been stored after harvest under cold conditions, and then optionally, appropriately treated with the above treatments. To prevent the quality deterioration of royal jelly during preparation steps, any of the above steps should desirable be carried out, usually, at temperatures lower than ambient temperature, preferably, 30°C or lower.

The cell activating agent in a solid form of the present invention is, for example, obtainable by mixing royal jelly and trehalose and optionally further mixing with another ingredients, and then subjecting the resulting mixture to conventional drying steps such as drying *in vacuo* or under reduced pressures or heating conditions. By using trehalose anhydride, such a solid cell activating agent according to the present invention can be obtained without employing conventional drying step: Admix crystalline or amorphous trehalose anhydride with royal jelly in an amount, usually, of at least 4-times, preferably, at least 8-times by weight of trehalose to the weight of the royal jelly as a raw royal jelly, and optionally mix with another ingredient(s), and then allow the resulting mixture to stand at temperatures not higher than ambient temperature, preferably, 30°C or lower, usually, for at least four hours, preferably, at least eight hours. The cell activating agent, prepared with trehalose anhydride without the need of any conventional drying step, has a feature of being specifically, advantageously stable in the cell activating action of royal jelly. If necessary, the agent in a solid form thus obtained can be processed into the desired form of a powder, tablet, film or sheet using pulverizes, tabletting machines, rolling machines, etc.; and optionally the resulting powers can be further injected into capsules for use.

As described above, the cell activating agent of the present invention *per se* is useful and can be arbitrarily used in the form of a composition combined with another ingredient(s). Thus, the present invention also provides such a composition. The composition according to the present invention usually contains one or more ingredients administrable to mammals including humans orally, percutaneously or externally, and are arbitrarily used, for example, in the fields of food products, cosmetics, and pharmaceuticals. Examples of the above ingredients include those which are usually used in the fields where the composition of the present invention is suitably used; water, alcohols, amylaceous substances, proteins, amino acids, fibers, saccharides, lipids, fatty acids, vitamins, minerals, flavors, colors, sweeteners, seasonings, spices, antiseptics, emulsifiers, and surfactants.

Although the composition of the present invention should not be specifically restricted to, preferable ones are those which can be used at temperatures of not higher than ambient temperature, preferably, 30°C or lower, in view of more effective exertion of the cell activating action by the composition as the cell activating agent of the present invention. Preferable examples in the form of a food are, for example, frozen desserts such as an ice cream, ice candy, and sherbet; syrups such as a "*korimitsu*" (a sugar syrup for shaved ice); spreads and pastes such as a butter cream, custard cream, flour paste, peanut paste, and fruit paste; Western cakes such as a chocolate, jelly, candy, gummy jelly, caramel, chewing gum, pudding, cream puff, and sponge cake; processed fruit and vegetables such as a jam, marmalade, "*syrup-zuke*" (a fruit pickle), and "*toka*" (conserves); Japanese confectioneries such as a "*manju*" (a bun with a bean-jam), "*uiro*" (a sweet rice jelly), "*an*" (a bean jam), "*yokan*" (a sweet jelly of beans), "*mizu-yokan*" (a soft adzuki-bean jelly), *pao de Castella,* and "*amedama*" (a Japanese toffee); and seasonings such as a soy sauce, powdered soy sauce, "*miso*", "*funmatsu-miso*" (a powdered *miso*), mayonnaise, dressing, vinegar, *"sanbai-zu"* (a sauce of sugar, soy sauce and vinegar), table sugar, and coffee sugar. Preferable examples of beverages are, for example, alcoholic beverages such as a synthetic sake, fermented liquor, fruit wine, and other foreign liquors; and soft drinks such as a juice, mineral beverage, carbonated beverage, sour milk beverage, beverage containing lactic acid bacteria, sport drink, supplemental drink, tea, black tea, oolong tea, coffee, and cocoa. Preferable examples of cosmetics are, for example, those in the form of a lotion or cream such as a basic skin care, cleansing cosmetic, bath cosmetic, hair cosmetic, sun burn or sun burn-preventing cosmetic, makeup cosmetic, hair restorer, and hair tonic. To produce the above-mentioned compositions according to the present invention, either of the cell activating agent of the present invention or both of royal jelly and trehalose can be added to materials for the compositions at an appropriate timing during processings of the desired products by using techniques according to conventional preparations. The timing for addition should not be specifically restricted to, however, in the case of producing the desired products through a heating step, the cell activating agent should preferably be added to the contents after being passed through the heating step and then cooled to a temperature not higher than ambient temperature, preferably, 30°C or lower, to prevent the reduction of the level of cell activating action during the processings. The above composition according to the present invention contains, usually, 0.001 to 20% by weight, preferably, 0.01 to 10% by weight of the cell activating agent of the present invention to the weight of a final product.

Since the composition according to the present invention exerts the cell activating action by royal jelly and has a stabilized action, the composition effectively exerts the desired cell activating action in living bodies when daily applied thereunto similarly as conventional products, resulting in an augmentation of biological resistivity, acceleration of the improvement of disorder of health condition, and maintenance of health condition. Thus, the composition of the present invention is distinctively useful as cosmetics and food products including beverages for the maintenance/promotion of the health. Also, in the case of using the composition as a cosmetic for applying to the skin including scalps, the composition effectively prevents and improves the symptom of dermatological diseases, prevents/improves wrinkles, and improves hair growth and regeneration.

The following experiments and examples disclose the present invention:

Experiment 1

Influence of saccharides on the stabilization of cell activating action

Experiment 1-1

Preparation of anhydrous trehalose

Using a jacketed rotary-vacuum-dryer, "TREHA®", a hydrous crystalline trehalose powder commercialized by Hayashibara Shoji Co., Okayama, Japan, was dried at 90°C for about seven hours under a reduced pressure of 300-350 mmHg. Thereafter, the resulting product was cooled to ambient temperature and then the inner pressure of the dryer was increased to normal pressure, followed by collecting the resultant product to obtain anhydrous trehalose.

Experiment 1-2

Preparation of cell activating agent

In this experiment, an unprocessed royal jelly, as a royal jelly, having 67% moisture by weight and being preserved at -20°C, from Brazil (abbreviated as "raw royal jelly") was thawed at ambient temperature in use and then promptly distributed by small portions in the desired amount.

Under 25°C, to one part by weight of the raw royal jelly were added four parts by weight of anhydrous trehalose obtained by the method in Experiment 1-1, followed by sufficiently mixing the mixture. The resulting mixture was dried overnight under a reduced pressure and pulverized by a pulverizer into a powder as the cell activating agent of the present invention (designated as "test agent 1" hereinafter).

Except for using a soluble starch in place of the anhydrous trehalose, another powder as reference agent 1 was prepared similarly as in the test agent 1. The other powder as reference powder 2 was prepared similarly as in the test agent 1 except for using anhydrous maltose in place of the anhydrous trehalose. In addition, a fresh preparation of the royal jelly was dried overnight at ambient temperature under a reduced pressure and pulverized by a pulverizer into a powder for reference agent 3.

Experiment 1-3

Stabilization of the action of prolonging cell survival

The following experiment examined the action of prolonging cell survival by raw royal jelly on rat liver cells in an in vitro culture system, the storage stability of the action by raw royal jelly, and the influence of coexisting saccharides on the storage stability of the jelly.

The test agent 1 and the reference agents 1 to 3, just after preparation by the method in Experiment 1-2, were respectively placed in brown glass bottles and then allowed to stand in a thermostatic chamber controlled at 25°C. A royal jelly just after thawing was placed in another brown glass bottle which was then placed in the thermostatic chamber similarly as above (designated as "thermostatically-preserved raw royal jelly" hereinafter). During a period of time up to 20 days from the day 0 received with the above treatment, the test agent 1 and the reference agents 1 to 3 were prepared daily and, just after the preparations, allowed to stand in the thermostatic chamber similarly as above, while a fresh preparation of raw royal jelly was thawed and allowed to stand in the thermostatic chamber similarly as above.

L-15 Medium supplemented with 20 µg/ml calf transferrin, 20 µM ethanol amine, 2.5x10$^{-8}$ M sodium selenite, 1x10$^{-6}$ M insulin, 1x10$^{-5}$ M dexamethasone, 100 µg/ml penicillin G, 100 µg/ml streptomycin, 25 ng/ml aprotinin, and 2 mg/ml sodium hydrogencarbonate was prepared as a basic culture medium for culturing liver cells *in vitro* (designated as "serum-free L-15 medium" hereinafter).

Either of the specimens, i.e., the test agent 1, the reference agents 1 to 3, and the thermostatically-preserved raw royal jelly whose storage period had reached 30 days, was added to serum-free L-15 medium to give a concentration of 10 mg/ml in terms of the weight of raw royal jelly for use as a test medium. Another medium was prepared by adding raw royal jelly just after thawing to serum-free L-15 medium to give a concentration of 10 mg/ml in terms of the weight of raw royal jelly for use as a positive control. Serum-free L-15 medium was used as a negative control.

A liver was extracted from a Wister rat, weighing about 200 g, and homogenized in a usual manner. The disruptant was treated with collagenase in a conventional manner to obtain liver cells. The liver cells thus obtained were suspended in the above test and control media into 2.5x10$^5$ cells/ml suspensions. Two milliliter aliquots of respective suspensions were transferred to plastic petri dishes, 35 mm in inner diameter, and incubated in a 5% $CO_2$ incubator at 37°C to culture the liver cells. At 24-hour intervals after the initiation of culture, the medium in each petri dish was replaced with a fresh preparation of each test or control medium, which had been prepared into a medium having the same composition as that of the test or the control medium using either the test specimen or the raw royal jelly just after thawing which had had been preserved for 30 days in the thermostatic chamber, followed by counting the number of total viable cells per petri dish in a usual manner. Each culture using the same composition was carried out in a triplicate manner.

In every culture system, the proliferation level of liver cells after 24-hour culture reached about 2-time cells of that of the culture initiation, and then the number of viable cells in each culture system tended to show different dynamics. In each culture system, the percentage (%) of the viable cells after 480-hour culturing to that of the 24-hour culture was calculated, followed by calculating the mean value of the percentage (%) for each system. The percentage (%) of viable cells was about 45% for the negative control, while it was about 90% for the positive control. Based on the result, it was confirmed that a fresh preparation of raw royal jelly had a distinct action of prolonging cell survival. When cultured with a test medium prepared with either of raw royal jelly or its processed products, i.e., the test agent 1 and the reference agents 1 to 3 which had been preserved under the prescribed conditions, each culture system showed a different viability of cells. The data indicated that the presence or absence of or the kind of coexisting saccharides influenced on the remaining cell viability, depending on the residual percentage (%) of the action of prolonging cell survival after preservation.

Based on the cell viability in each test medium, the residual percentage (%) of the action of prolonging cell survival was calculated by the equation below. The results on each test medium were tabulated in Table 1.

$$A\,(\%) = \frac{(P\text{-}N) - (B\text{-}N)}{(P - N)} \times 100$$

Note: The symbol "A" means a residual percentage (%) of the action of prolonging cell survival after preservation of raw royal jelly; "B", a percentage (%) of viable cells in each test medium; "P", a mean value of the percentages (%) of viable cells in the positive control; and "N", a mean value of the percentages (%) of viable cells in the negative control.

Table 1

| Sample | Remarks | Residual level* of the action of prolonging cell survival after preservation at 25°C for 30 days |
|---|---|---|
| Test agent 1 | Raw RJ + Trehalose | ++ |
| Reference agent 1 | Raw RJ + Starch | ± |
| Reference agent 2 | Raw RJ + Maltose | ± |
| Reference agent 3 | Dried raw RJ | - |
| Raw RJ preserved at constant temperature | - | - |
| Note : The symbol "*" means a residual level of the action of prolonging cell survival, where the symbols "++", "+", "±" and "-" mean 80% or more but 100% or lower, 60% or more but less than 80%, 40% or more but less than 60%, and 0% or more but less than 40%. The expression of "Raw RJ" means raw royal jelly. |||

As shown in Table 1, just after thawing, the raw royal jelly which had been preserved at 25°C for 30 days showed a distinctively lowered action of prolonging cell survival, while in the case with trehalose as in the test agent 1, even after the preservation under the same conditions as above, it showed substantially the same level of the action of prolonging cell survival as in the case of the raw royal jelly just after thawing. In the cases (reference agents 1 and 2) of incorporating either a soluble starch or anhydrous maltose into raw royal jelly, the action of prolonging cell survival was observed to some extent but the level of which was lower than that with trehalose. In addition, in the case (reference agent 3) of using a raw royal jelly prepared by direct drying, the residual action of prolonging cell survival was not so significant. These phenomena would had been induced as a result of the reduction of the action in the drying step of raw royal jelly rather than the poor storage stability of a dried raw royal jelly. These results indicate that trehalose distinctively augments the stability of cell activating action of royal jelly.

Experiment 2

Influence of the amount of trehalose on the stabilization of cell activating action

Under 25°C conditions, the raw royal jell used in Experiment 1 and anhydrous trehalose obtained by the method in Experiment 1-1 were mixed in a ratio as shown in Table 2 below, and then sufficiently mixed together, and dried at ambient temperature overnight under a reduced pressure, followed by pulverizing the resulting dried product into a powder.

In accordance with the method in Experiment 1-3, the above powder was preserved at 25°C for 30 days and then subjected to an experiment for confirming the action of prolonging cell survival of rat liver cells, where an L-15 medium as a positive control supplemented with a raw royal jelly just after thawing, and a serum-free L-15 medium as a negative control were employed. The amount of royal jelly in each test medium was controlled to give 10 mg/ml in this experiment in terms of the weight of raw royal jelly. In accordance with Experiment 1, the results of the residual levels of the action of prolonging cell survival with each of the post preserved samples were tabulated in Table 2.

Table 2

| Ratio (part by weight) | | Residual level* of the action of prolonging cell survival after the preservation at 25°C for 30 days |
|---|---|---|
| Raw royal jelly | Anhydrous trehalose | |
| 1 | 0 | - |
| 1 | 0.02 | - |
| 1 | 0.05 | ± |
| 1 | 0.1 | ± |
| 1 | 0.2 | + |
| 1 | 0.5 | + |
| 1 | 1 | + |
| 1 | 2 | + + |
| 1 | 5 | + + |

Note: The symbol "*" means the residual level of the action of prolonging cell survival, where the symbols "++", "+", "±" and "-" mean 80% or more but 100% or lower, 60% or more but less than 80%, 40% or more but less than 60%, and 0% or more but less than 40%.

As shown in Table 2, the stabilization effect by trehalose on the action of prolonging cell survival was observed when incorporated into the raw royal jelly in an amount of at least 0.05 time by weight of that of the raw royal jelly, particularly, the effect became distinctive when the incorporation ratio was 0.2-time or more and reached roughly a maximum level when the ratio was 2-times or more. These results indicate that the weight ratio of trehalose, in terms of its anhydrous form, to royal jelly is preferably at least 0.2-time by weight, more preferably, at least 2-times by weight, based on the weight of raw royal jelly.

As not shown the data, a panel test confirmed that the higher the stability of the specimens tested in this experiment, i.e., the more the content of anhydrous trehalose in the specimens, the more they exhibit a satisfactory taste than raw royal jelly. From a viewpoint of expression of the action of prolonging cell survival by royal jelly, the higher the content of royal jelly, the more preferably the cell activating agent of the present invention is used. Considering conventional doses of royal jelly for humans, at least one percent (%), preferably, at least two percent (%) by weight of a royal jelly, in terms of the weight of a raw royal jelly, to the total amount. Based on these, the cell activating agent of the present invention which comprises at least one percent (%), preferably, at least two percent (%) by weight of a royal jelly, in terms of the weight of a raw royal jelly, to the total amount; and trehalose, in terms of its anhydrous form, in an amount of at least 0.2-time, preferably, at least 2-times by weight of the royal jelly, has features that it satisfactorily stabilizes the action of prolonging cell survival as one of the cell activating actions, where the action is distinctive, and that it has a pleasant taste. Considering the case of incorporating ingredients other than the royal jelly and trehalose, the ratio of trehalose, in terms of its anhydrous form, to the royal jelly is usually not higher than 50-times, preferably, not higher than 25-times, based on the weight of raw royal jelly.

Experiment 3

Influence of trehalose on the activation of function of spermary by royal jelly

Just after the preparation of the test agent 1 in Experiment 1-2, as a preferred embodiment of the cell activating agent of the present invention, it was mixed with a conventional powdery feed for experimental animals to obtain a powdery feed comprising 50 ppm or 500 ppm royal jelly, in terms of the weight of raw royal jelly, to the total weight (designated as "test feed" hereinafter). Also, another feed (designated as "control feed" hereinafter) was prepared by replacing the test agent 1 in the test feed, containing 500 ppm royal jelly, with the anhydrous trehalose prepared in Experiment 1-1. Thirty male hamsters, aged 26-weeks, were fed on the control feed for one week, and then grouped into three groups consisting of 10 heads per group. The hamsters in the groups 1 to 3 were bred for 12 weeks while allowing to freely intake the control feed, the test feed with 50 ppm royal jelly, and the test feed with 500 ppm royal jelly, respectively, through a commercialized feeder for powdery feeds.

During the breeding, the amount of feed intake and the body weight of each hamster were weighed daily.

After completion of the breeding, the function of spermary in each hamster was examined by analyzing a specimen from each spermary as shown below: The right spermary of each hamster was extracted and fixed with a 15% neutral buffered formalin solution in a usual manner to obtain a specimen of spermary tissue. Each specimen was stained with hematoxylin-eosin, and seminiferous tubules were macroscopically observed and examined histologically. In each specimen, randomly selected five spots were microscopically observed with a magnification of 100 to count the number of seminiferous tubules within a field, followed by grouping the observed seminiferous tubules into a group with regressive change in the deterioration/disappearance of sexual cells or in the cavity of seminiferous tubules, and another group with rigidly sustained tissue structure and active sperm production. For each specimen, a mean average of the percentages (%) of sperm-production-active-seminiferous-tubules present in the total seminiferous tubules was determined, and the values for each group were averaged.

Over the above breeding period, a daily feed intake in each hamster was within about 5% by weight of the body weight, and there was no difference among the groups tested. No change on the body weight in each animal was found among the groups. The results on histological analysis of spermary tissues are tabulated in Table 3.

Table 3

| Group | Feed | Percentage (%) of seminiferous tubules active in sperm production |
|---|---|---|
| 1 | Control feed free of RJ | About 15% |
| 2 | Test feed with 50 ppm RJ | About 25% |
| 3 | Test feed with 500 ppm RJ | About 50% |
| Note: The symbol "RJ" means royal jelly. | | |

As shown in Table 3, the percentage (%) of seminiferous tubules active in sperm production increased depending on the intake of royal jelly. Under microscopic observation, the group administered with the test feed containing the test agent 1, particularly, that administered with the test feed containing 500 ppm royal jelly resulted in a clear observation of an increased number of sperm formation as compared with the group fed on the control feed.

Experiments similar to the above were conducted using, in place of the test agent 1 just after preparation as used in the above experiment, the reference agents 1 and 2 which had been prepared by the method in Experiment 1-2 and then preserved at 25°C for 30 days under light-shielded conditions according to the method in Experiment 1-3, and a raw royal jelly which had been preserved at a constant temperature of 25°C for 30 days under light-shielded conditions according to the method in Experiment 1-3. As a result, substantially the same result as above was obtained when experimented with the test agent 1 after preservation. While, in the case of using the reference agent 2 after preservation and the raw royal jelly after constant temperature preservation, the percentage (%) of seminiferous tubules active in sperm production did not tend to clearly increase depending on the intake of the raw royal jelly. In spite of the intake of raw royal jelly, there were found many hamsters that showed substantially the same level of percentage (%) as that of those in the control group.

The above data indicate that the addition of trehalose distinctively stabilizes the action of activating the function of spermary by royal jelly.

Experiment 4

Influence of trehalose on the action of prolonging the life-span of mice by royal jelly

Just after the preparation by the method in Experiment 1-2, the test agent 1, as an example of the cell activating agent of the present invention, was mixed with a conventional powdery feed for experimental animals to obtain a powdery feed comprising 50 ppm or 500 ppm royal jelly, in terms of raw royal jelly, to the total weight of feed (designated as "test feed" hereinafter). Another feed (designated as "control feed" hereinafter) was prepared by replacing the test agent 1, as the test feed containing 500 ppm royal jelly, with an equal amount of the anhydrous trehalose prepared in Experiment 1-1. Thirty male C3H/HeJ mice, aged 7-weeks, were grouped into three groups consisting of 10 heads per group. The mice in the groups 1 to 3 were bred while allowing them to freely intake the control feed, the test feed with 50 ppm royal jelly, and the test feed with 500 ppm royal jelly, respectively, through a commercialized feeder for powdery feeds. The daily intake of each feed in each alive animal was substantially the same level as that of those with a feed in general, and no difference was found among the groups tested.

Among the mice in the first group bred on the control feed, there begun to observe a death mouse at the age of 32-weeks, and then resulted in 3-death mice at the age of 42-weeks. While, all the mice in the second and the third groups bred on the feed with the test agent 1 as the cell activating agent of the present invention were alive even at the age of 42-weeks.

In place of the test agent 1 just after preparation as used in the above experiment, experiments similar to the above were conducted using the test agent 1 and the reference agent 2 which had been prepared by the method in Experiment 1-2 and preserved at 25°C for 30 days under light-shielded conditions according to the method in Experiment 1-3, and a raw royal jelly which had been preserved at a constant temperature of 25°C for 30 days under light-shielded conditions according to the method in Experiment 1-3. As a result, all the mice in the group bred on the test agent 1 after preservation were alive at the age of 42-weeks similarly as above. While, the mice in the groups bred on either the reference agent 2 after preservation or the raw royal jelly preserved at the constant temperature resulted in showing a survival curve similar to that of the group bred on the control feed, i.e., two or three mice were dead at the age of 42-weeks. These results indicate that the addition of trehalose to royal jelly stabilizes the action of prolonging murine life by royal jelly.

Example 1

Health food

Nine parts by weight of anhydrous trehalose, obtained by the method in Experiment 1-1, and one part by weight of a fresh preparation of the same raw royal jelly just after thawing as used in Experiment 1-2 were mixed to homogeneity under the condition of 25°C. The mixture was allowed to stand at 25°C overnight and pulverized by a pulverizer into a powder which was then passed through a mesh sieve with a 0.42 mm in diameter, followed by collecting the resulting powder to obtain a cell activating agent according to the present invention. Treatment in accordance with the method in Experiment 1 confirmed that the agent stably exhibited the action of prolonging cell survival even after the preservation at ambient temperature.

The agent was formed by a tabletting machine into a tablet, about 200 mg. The agent is a cell activating agent which stably exhibits a cell activating action even after the preservation at ambient temperature, and has easy handleability and strong effect. Since the agent has a mild sweetness and adequate acidity, it is useful as a daily health food.

Example 2

Health food

The following ingredients were mixed to homogeneity in the ratio as indicated below and, in accordance with Example 1, the mixture was processed into a cell activating agent in a powder form according to the present invention. After preparation, the agent was treated similarly as in Experiment 1 and confirmed that it showed a stable action of prolonging cell survival even after the preservation at ambient temperature.

| | |
|---|---|
| Anhydrous trehalose obtained by the method in Experiment 1-1 | 8.5 parts by weight |
| Raw royal jelly just after thawing in Experiment 1-2 | 0.5 part by weight |
| "αG HESPERIDIN PS", a product name of a glycosyl hesperidin commercialized by Hayashibara Shoji Co., Ltd., Okayama, Japan | 0.5 part by weight |
| "PULLULAN PF-20", a product name, commercialized by Hayashibara Shoji Co., Ltd., Okayama, Japan | 0.5 part by weight |

The above agent was shaped by a tabletting machine into a tablet, about 300 mg. The agent is a cell activating agent which stably exhibits a cell activating action even after the preservation at ambient temperature, and has easy handleability and strong effect. Since the agent has a mild sweetness and adequate acidity, it is useful as a daily health food.

Example 3

Health food

The following ingredients were mixed to homogeneity in the ratio as indicated below and, in accordance with Example 1, the mixture was prepared into a cell activating agent in a powder form according to the present invention. After preparation, the agent was treated similarly as in Experiment 1 and confirmed to exhibit a stable action of prolonging cell survival even after the preservation at ambient temperature.

| | |
|---|---|
| Anhydrous trehalose obtained by the method in Experiment 1-1 | 7.5 parts by weight |
| Raw royal jelly just after thawing in Experiment 1-2 | 1.0 part by weight |
| Maltitol | 1.3 parts by weight |
| L-Tryptophane | 0.2 part by weight |

The agent is a cell activating agent which stably exhibits a cell activating action even after the preservation at ambient temperature and has easy handleability and strong effect. Since the agent has a satisfactory taste due to its mild sweetness and adequate acidity, it is useful as a daily health food.

Example 4

Ice cream

Eighteen parts by weight of a raw cream containing about 46% by weight of oils and fats, seven parts by weight of a skim milk powder, 51 parts by weight of a whole milk, 10 parts by weight of sugar, four parts by weight of "NYUKA-OLIGO®" as a lactosucrose-containing powder, two parts by weight of pullulan, and two parts by weight of gum arabic were melted, kept at 70°C for 30 min for sterilization, and emulsified and dispersed by a homogenizer. Thereafter, the mixture was promptly cooled to 3 to 4°C and mixed with four parts by weight of a cell activating agent obtained by the method in Example 2, followed by aging the resulting mixture overnight and then freezing the aged mixture by a freezer into an ice cream.
The product is an ice cream which exhibits an adequate sweetness and elegant taste, has the action of activating cells, and maintains/promotes the health.

Example 5

*Amazake* (a sweet drink made from fermented rice)

To 10 parts by weight of milled rice was added water and boiled in a usual manner, and then the boiled rice was cooled to 55°C and mixed with 30 parts by weight koji, which had been prepared in a conventional manner, and 0.1 part by weight of salt. The mixture was kept at 50-55°C for eight hours, fed to a mixer and cooled to about 25°C, and then admixed with two parts by weight of a cell activating agent obtained by the method in Example 1, followed by injecting the resulting mixture into small bags to obtain the captioned product.
Since the product is an *amazake* which has a satisfactory color tint, flavorful taste, and elegance and exhibits a moderate cell activating action, it is useful as a beverage for maintaining/promoting the health.

Example 6

Health beverage

A composition was prepared with 500 parts by weight of anhydrous crystalline maltose, 100 parts by weight of the cell activating agent in Example 3, 190 parts by weight of an egg yolk powder, 200 parts by weight of a skim milk powder, 4.4 parts by weight of sodium chloride, 1.85 parts by weight of potassium chloride, four parts by weight of magnesium sulfate, 0.01 part by weight of thiamine, 0.1 part by weight of sodium ascorbate, 0.6 part by weight of vitamin E acetate, and 0.04 part by weight of nicotinamide. Twenty-five parts by weight of the composition were homogeneously dispersed and dissolved in 150 parts by weight of refined water, and 150 g aliquots of the resulting mixture were distributed to and sealed in brown bottles.

Since the product stably exhibits cell activating action and is supplemented with nutrients, it can be arbitrarily used as a health beverage for maintaining the health, promoting the growth, preventing diseases, promoting therapeutic treatments, and promoting the recovery of normal conditions from fatigue after sport. The product can be also used as an orally or intubationally administrable composition to animals such as domestic animals, as well as to humans.

Example 7

External dermatological cream

The following ingredients were mixed by heating in a usual manner according to the ratio as indicated below:

| | |
|---|---|
| Polyoxyethylene glyceryl monostearate | 2.0 parts by weight |
| Glyceryl monostearate, self-emulsifying | 5.0 parts by weight |
| Eicosanyl behenate | 1.0 part by weight |
| Liquid petrolatum | 1.9 parts by weight |
| Trimethylol propane trioctanate | 10.0 parts by weight |

To the resulting mixture were added the following ingredients except for the cell activating agent, and the resulting mixture was mixed and cooled to 30°C or lower. Thereafter, the mixture thus obtained was admixed with the cell activating agent in a ratio as indicated below and emulsified by a homogenizer into an external dermatological cream.

| | |
|---|---|
| 1,3-Butylene glycol | 5.0 parts by weight |
| Sodium lactate solution | 10.0 parts by weight |
| Methyl *p*-hydroxybenzoate | 0.1 part by weight |
| Peach leaf extract | 1.5 parts by weight |
| Refined water | 62.2 parts by weight |
| Cell activating agent obtained by the method in Example 1 | 1.0 part by weight |

Since the cream thus obtained exhibits a satisfactory humectancy and imparts activity to skin cells, it will be advantageously useful as a basic skin care for keeping fresh and youthful skin.

POSSIBILITY OF INDUSTRIAL APPLICABILITY

As described above, the present invention was made based on the complete self-finding that the cell activating agent, comprising royal jelly and trehalose, exerts a distinct cell activating action inherent to raw royal jelly and effectively stabilizes the action during storage. Since the cell activating agent has no serious side effect and imparts a mild sweetness due to the coexistence of trehalose, it is easily and pleasantly used for humans and mammals to maintain/promote their health. By combining with other ingredient(s), the cell activating agent of the present invention having these advantageous features can be used as compositions such as foods, beverages, and cosmetics.

The present invention with such outstanding effects and functions is a significant invention that will greatly contribute to the art.

**Claims**

1. A cell activating agent comprising a royal jelly and $\alpha,\alpha$-trehalose.

2. The agent of claim 1, which further contains one or more antioxidants.

3. The agent of claim 2, wherein said antioxidants are flavonoids, polyphenols, vitamin E, and vitamin C.

4. The agent of any one of claims 1 to 3, which further contains a sugar alcohol and/or a polysaccharide thickener.

5. The agent of claim 4, wherein said sugar alcohol is maltitol and/or said polysaccharide thickener is pullulan.

6. The agent of any one of claims 1 to 5, which is in the form of a solid.

7. The agent of any one of claims 1 to 6, which contains said royal jelly, in terms of a raw royal jelly, in an amount of one percent (%) by weight; and said $\alpha,\alpha$-trehalose, in terms of an anhydrous form, in an amount of 0.2 to 50 times by weight of said royal jelly.

8. The agent of any one of claims 1 to 7, which is in the form of a tonic, health food, or health supplement.

9. A process for producing the agent of any one of claims 1 to 8, which comprises a step of mixing royal jelly with trehalose.

10. The process of claim 9, wherein said $\alpha,\alpha$-trehalose is in an anhydrous form.

11. A composition comprising the cell activating agent as claimed in any one of claims 1 to 8.

12. A composition comprising royal jelly and $\alpha,\alpha$-trehalose.

13. The composition of claim 11 or 12, which is in the form of a food, beverage, or cosmetic.

## INTERNATIONAL SEARCH REPORT

| | International application No. |
|---|---|
| | PCT/JP01/01045 |

A. CLASSIFICATION OF SUBJECT MATTER
    Int.Cl$^7$   A61K35/64, 31/7016, 7/00, 7/48, A61P43/00, 7/00, A23L1/29, 1/076

According to International Patent Classification (IPC) or to both national classification and IPC

B. FIELDS SEARCHED

Minimum documentation searched (classification system followed by classification symbols)
    Int.Cl$^7$   A61K35/64, 31/7016, 7/00, 7/48, A23L1/29, 1/076

Documentation searched other than minimum documentation to the extent that such documents are included in the fields searched

Electronic data base consulted during the international search (name of data base and, where practicable, search terms used)
    WPI,JOIS

C. DOCUMENTS CONSIDERED TO BE RELEVANT

| Category* | Citation of document, with indication, where appropriate, of the relevant passages | Relevant to claim No. |
|---|---|---|
| Y | EP, 600730, A1 (KABUSHIKI KAISHA HAYASHIBARA SEIBUTSU KAGAKU KENKYUJO), 08 June, 1994 (08.06.94), whole document, especially Claims; p.2 line 37; p.5, line 42; p.6 lines from 1 to 9 & JP, 6-170221, A   & US, 5543513, A | 1-13 |
| Y | JP, 11-32705, A (YAKULT HONSHA CO., LTD.), 09 February, 1999 (09.02.99), Full text; especially, Claims; example   (Family: none) | 1-3,6-13 |
| Y | JP, 60-37942, A (Sanraku Inc.), 27 February, 1985 (27.02.85), Full text; especially, p.2, lower right column, line 4 (Family: none) | 1-13 |
| Y | Yoshimasa HAYASHI "Royal Gelly no Tokusei to Ouyou" Fragrance Journal, Extra issue, No.4(1983) pp.159~161, Full text | 1-13 |
| Y | JP, 10-279463, A (The Nisshin Oil Mills, Ltd.), 20 October, 1998 (20.10.98), | 1-13 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 April, 2001 (10.04.01) | 24 April, 2001 (24.04.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

14

## INTERNATIONAL SEARCH REPORT

| | International application No. |
|---|---|
| | PCT/JP01/01045 |

C (Continuation).   DOCUMENTS CONSIDERED TO BE RELEVANT

| Category* | Citation of document, with indication, where appropriate, of the relevant passages | Relevant to claim No. |
|---|---|---|
| | Claim 5   (Family: none) | |
| Y | JP, 10-95704, A (KOSE Corporation), 14 April, 1998 (14.04.98), Claim 5   (Family: none) | 1-13 |
| PX PA | JP, 2000-342231, A (Kanebo, LTD.), 12 December, 2000 (12.12.00), Par. No. [0032]   (Family: none) | 1-3,6-13 4,5 |

Form PCT/ISA/210 (continuation of second sheet) (July 1992)